(19) Europäisches Patentamt
European Patent Office
Office européen des brevets

(11) **EP 3 454 776 B1**

(12) **EUROPÄISCHE PATENTSCHRIFT**

(45) Veröffentlichungstag und Bekanntmachung des Hinweises auf die Patenterteilung:
**24.06.2020 Patentblatt 2020/26**

(21) Anmeldenummer: **17723059.6**

(22) Anmeldetag: **09.05.2017**

(51) Int Cl.:
***A61C 9/00*** *(2006.01)*

(86) Internationale Anmeldenummer:
**PCT/EP2017/061047**

(87) Internationale Veröffentlichungsnummer:
**WO 2017/194536 (16.11.2017 Gazette 2017/46)**

(54) **COMPUTERIMPLEMENTIERTES VERFAHREN ZUR FESTLEGUNG EINER ZAHNAUFSTELLUNG**

COMPUTER IMPLEMENTED METHOD FOR DEFINING A DENTAL ARRANGEMENT

PROCÉDÉ IMPLÉMENTÉ PAR ORDINATEUR POUR ÉTABLIR UN ARRANGEMENT DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.05.2016 EP 16169206**

(43) Veröffentlichungstag der Anmeldung:
**20.03.2019 Patentblatt 2019/12**

(73) Patentinhaber: **Vita Zahnfabrik H. Rauter GmbH & Co. KG**
**79713 Bad Säckingen (DE)**

(72) Erfinder:
• **CHRISTEN, Urban**
**79713 Bad Säckingen (DE)**
• **KERSCHENSTEINER, Eva**
**79713 Bad Säckingen (DE)**
• **EGLE, Franz**
**79713 Bad Säckingen (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
WO-A1-2015/169910    JP-A- 2005 168 518
US-A1- 2010 151 417

**Beschreibung**

Die Erfindung betrifft ein Verfahren zur Festlegung einer Zahnaufstellung für die Herstellung von Zahnvoll- und/oder Zahnteilprothesen.

Bei der Versorgung von Patienten mit dentalen Teil- oder Vollprothesen ist heutzutage ein hoher Standard festzustellen. Der Zahnarzt bereitet die zur Versorgung vorgesehene Situation am Patienten vor, während die Zahn(teil)prothese üblicherweise in einem externen Labor oder einem Praxislabor von einem Zahntechniker nach den Vorgaben des Zahnarztes hergestellt wird. Die Qualität der Prothese ist dabei in großem Maße abhängig von der handwerklichen Geschicklichkeit des Zahntechnikers, der die Vorgaben des Zahnarztes in der Herstellung der Prothese berücksichtigen muss, sowie der Qualität dieser Vorgaben.

Des Weiteren ist die Vorbereitung des prothetisch zu versorgenden Patienten für diesen oftmals mit Unannehmlichkeiten verbunden. Erwähnt seien in diesem Zusammenhang beispielsweise verschiedene Sitzungen beim behandelnden Zahnarzt, in denen Abformungen der zu versorgenden Situation vorgenommen werden müssen, die dem Zahntechniker als Negativmodelle dienen. Die sich anschließenden weiteren Abformungen sind zeitaufwendig und oftmals der Grund für fehlerhafte Ausführung der prothetischen Versorgung. Typischerweise sind zwei oder drei Abformungssitzungen erforderlich, bevor der Zahntechniker mit der eigentlichen Herstellung der Prothese beginnen kann.

Üblicherweise erfolgt die Zahnaufstellung durch den Zahntechniker zahnweise. Verschiedene Aufstellungsverfahren wie beispielsweise die Aufstellung nach Staub (s. z.B. US 2010/151417 A1) helfen dabei dem Zahntechniker bei der Erstellung einer geeigneten Zahnaufstellung. Nachteilig hierbei ist jedoch, dass eine optimale Passform der einzelnen Zähne der Zahnaufstellung zueinander gefunden muss, wobei gleichzeitig die anatomischen Gegebenheiten beim Patienten berücksichtigt werden müssen, als auch das Zusammenspiel zwischen den Zähnen des Oberkiefers und des Unterkiefers. Auf Grund der Verwendung einzelner Zähne als Ausgangspunkt der Zahnaufstellung ergibt sich eine große Anzahl an Realisierungsmöglichkeiten und damit einhergehend eine große Anzahl an Fehlerquellen, die zu einer fehlerhaften Zahnaufstellung führen und damit beim Pateinten Beschwerden auslösen können.

WO 2011/066895 A1 betrifft ein Verfahren für die automatisierte Herstellung von Zahnersatz, umfassend die Schritte Bereitstellen eines digitalen Datensatzes der herzustellenden individuellen Prothese, digitale Trennung des Modells in Zahnbogen und Zahnfleisch, Herstellung des Zahnbogens aus Keramik und Kunststoff mittels Frästechnik oder Herstellung der Prothesenbasis durch generative oder abtragende Methoden aus überwiegend (Meth)-Acrylat-basierten Kunststoffen, den Anschluss des Zahnbogens und das Zahnfleisch durch Kleben oder Verbinden oder eine Kombination von Kleben und Fügen.

EP 1 864 627 A2 offenbart ein Verfahren zur Herstellung von Zahnprothesen nach einem digitalisierten, virtuellen Modell, welches die Kiefersituation wiedergibt, mit den Schritten einer digitalen Erfassung der Kiefersituation und-relationen, digitalen Aufstellung (automatisch als Option), Erzeugung einer geteilten Negativform (Rapid Manufacturing) aus den Daten der digitalen Zahnaufstellung, Einstecken der Konfektionszähne/ konfektionierten Zahneinheiten in die geöffneten Negativformen, Schließen der Negativformen, und Ausfüllen der verbliebenen Hohlräume mit Prothesenkunststoff.

EP 1 444 965 A2 betrifft ein Verfahren zur Herstellung von Zahnersatz, mit den Schritten, Aufnahme und Digitalisierung (Einscannen) der 3-dimensionalen, anatomischen Verhältnisse in der Mundhöhle, gegebenenfalls Aufnahme und Digitalisierung (Einscannen) der 3-dimensionalen Daten von Bissschablonen incl. Bisswällen, gegebenenfalls Aufnahme der Kieferdaten, die normalerweise am Patienten zur Einstellung des Artikulators genommen werden, erhaltenen Verarbeitung des Datensatzes in der Weise, dass die relevanten anatomischen Strukturen für eine virtuelle Zahnaufstellung festliegen und ein virtuelles Modell als Datensatz erhalten wird, gefolgt von der Auswahl der 3-D Datensätze konfektionierter, vorher eingescannter Zähne aus einem weiteren Datensatz, virtuelle Aufstellung der Zähne in das virtuelle Modell als zweiten Datensatz sowie entweder gefolgt von einem Übertragen der virtuellen Aufstellung auf das Modell durch entweder Positionierschablone (z.B. gefräst oder Rapid Prototyped) oder direkte Aufstellung der konfektionierten Zähne auf das Modell, Fixierung der Zähne auf dem Modell, Anbringen der Prothesenbasis oder in einer anderen Alternative, gefolgt von einer direkten Herstellung der Prothesenbasis - nach den Daten der virtuellen Zahnaufstellung - mit Positionierhilfen für die endgültige korrekte Positionierung und Fixierung der konfektionierten Zähne. Dieses Verfahren zur Herstellung von Zahnersatz ist äußerst aufwendig. Insbesondere ist der Aufwand hoch um einen präzisen Zahnersatz herzustellen.

US 2010/151417 A1 bezieht sich auf ein Verfahren und System zur Planung von zahnärztlichen Restaurationsverfahren und zur Herstellung von Zahnersatz und/oder zahntechnischen Komponenten im Zusammenhang mit den zahnärztlichen Restaurationsverfahren.

Aufgabe der Erfindung ist es, ein Verfahren zur Festlegung einer Zahnaufstellung für die Herstellung von Zahnvollprothesen oder Teilprothesen zu schaffen, mit dem auf möglichst einfache Weise eine zufriedenstellende Auswahl der Zahnaufstellung erfolgen kann.

Die Lösung der Aufgabe erfolgt erfindungsgemäß durch die Merkmale des Anspruchs 1.

Zunächst erfolgt das Erfassen der oralen Patientensituation. Dies kann durch bekannte Verfahren beispielsweise durch Abformen und Bissnahme und/oder mittels digitaler Aufnahmen und gegebenenfalls Digitalisierung der oralen Patientensituation erfolgen. Beispielsweise beim Verwenden von Bissschablonen für den Ober- und den Unterkiefer erfolgt ein Abdruck der aktuellen Patientensituation. Diese kann digitalisiert werden, wobei das Negativ der Bissschablone die tatsächliche orale Patientensituation abbildet, so dass diese im Rechner abgebildet ist. Eine entsprechende Abbildung der oralen Patientensituation kann auch durch unmittelbares digitales Abtasten der Patientensituation erfolgen. Als Ergebnis ist stets die tatsächliche orale Patientensituation im Rechner abgebildet. Erfindungsgemäß erfolgt zunächst ein Bestimmen von Koordinaten definierter Basispunkte eines Unter- und/oder eines Oberkiefers. Dies erfolgt vorzugsweise mittels des im Rechner abgebildeten Unterkiefers- und/oder Oberkiefers, das heißt der abgebildeten oralen Patientensituation.

Auf Grundlage der koordinatendefinierten Basispunkte ist es möglich, für den Unterkiefer und/oder den Oberkiefer ein Transversalmaß und/oder eine skelettale Klasse und/oder eine Bissart zu ermitteln. Hierbei ist es insbesondere bevorzugt, dass insbesondere zwei und besonders bevorzugt drei dieser Merkmale möglichst detailliert bestimmt werden. Je mehr Merkmale und je detaillierter diese Merkmale bestimmt werden, desto qualitativ hochwertiger ist die Festlegung der Zahnaufstellung. Ein vollständiges und aufwendiges Vermessen der Mundhöhle des Patienten ist nicht erforderlich. Die Bestimmung der Merkmale erfolgt lediglich durch Erfassung einzelner Basispunkte. Hierdurch ist das Verfahren schnell und weniger aufwendig.

Im nächsten Schritt erfolgt sodann eine Auswahl einer passenden Zahnaufstellung auf Grundlage der ermittelten Daten, das heißt auf Grundlage des ermittelten Transversalmaßes und/oder der ermittelten skelettalen Klasse und/oder der ermittelten Bissart. Die Zahnaufstellung kann dabei ausgewählt werden aus einer Vielzahl möglicher Zahnaufstellungen. Hierbei ist es insbesondere nicht mehr erforderlich einzelne Zähne in einer Zahnaufstellung zusammenzufügen. Vielmehr wird die passende Zahnaufstellung, welche bereits optimal zueinander passende künstliche Zähne enthält, ausgewählt anhand der ermittelten Daten. Ein Anpassen der künstlichen Zähne zueinander innerhalb der Zahnaufstellung und die damit verbundenen Fehlerquellen entfallen hierdurch. Insbesondere wird auch die Bissfunktion von Ober- und Unterkiefer zueinander berücksichtigt, so dass mit Auswahl der passenden Zahnaufstellung bereits eine optimale Interaktion zwischen den Zähnen des Oberkiefers und des Unterkiefers gewährleistet ist, ohne dass weitreichende und aufwendige Anpassungen erforderliche werden. Insbesondere ist es durch die algorithmische Auswahl der passenden Zahnaufstellung möglich die Anzahl erforderlicher Sitzungen des Patienten zu Anpassung der Zahn(teil)prothese zu reduzieren und somit den Aufwand für Patient und Zahnarzt zu reduzieren. Gleichzeitig ist es hierdurch möglich schneller Zahnersatz bereitzustellen.

Die ermittelte Zahnaufstellung und somit die festgelegten künstlichen Zähne werden sodann in einem weiteren Schritt in eine angefertigte Prothesenbasis eingesetzt. Die Herstellung der Prothesenbasis kann hierbei beispielsweise durch ein CAD/CAM-System wie in WO 2015/078701 beschrieben erfolgen.

Erfindungsgemäß wird die passende Zahnaufstellung ausgewählt aus einer Vielzahl von vorherbestimmten Zahnaufstellungen. Diese können beispielsweise in einer Datenbank hinterlegt sein. Hierdurch wird das Verfahren zur Erstellung einer Zahn(teil)prothese deutlich vereinfacht und insbesondere beschleunigt, da nicht mehr aufwendig einzelne Zähne herausgesucht werden müssen, um diese dann zu einer Zahnaufstellung zusammen zu führen.

In besonders bevorzugter Ausführungsform der Erfindung erfolgt das Bestimmen des Transversalmaßes als Abstand der tiefsten Stellen des Kieferkamms. Dies erfolgt vorzugsweise aufgrund der im Rechner abgebildeten tatsächlichen oralen Patientensituation des Ober- und/oder Unterkiefers. Hierzu ist es insbesondere bevorzugt, dass die Basispunkte die am weitesten mesial gelegenen Punkte der retromolaren Dreiecke auf der rechten und linken Seite des Unterkiefers umfassen. Das Transversalmaß kann sodann vorzugsweise aus dem Abstand zwischen den beiden am weitesten mesial gelegenen Punkten erfolgen.

Das Transversalmaß kann somit vorzugsweise ermittelt werden durch

$$\frac{rd - tmm}{2} = y$$

wobei

$t_{mm}$ das Transversalmaß
$r_d$ die Strecken zwischen den mesialen Punkten des retromolaren Dreiecks und
$y$ der Umrechnungsfaktor ist.

In bevorzugter Ausführungsform werden die Koordinaten der entsprechenden Basispunkte, das heißt zur Ermittlung des Transversalmaßes die Koordinaten der beiden mesialsten Punkte der retromolaren Dreiecke ermittelt. Der

Abstand $d_r$ berechnet sich bei der Verwendung von kartesischen Koordinaten aus

$$d_r(rdr; rdl) = \sqrt{(x_{rdr} + x_{rdl})^2 + (y_{rdr} - y_{rdl})^2 + (z_{rdr} - z_{rdl})^2} \quad (Gl.\ 2)$$

wobei x, y, z, die Koordinaten des linken (l) bzw. des rechten (r) mesialsten Punktes sind.

Zusätzlich oder anstelle der Bestimmung des Transversalmaßes erfolgt in bevorzugter Ausführungsform ein Bestimmen der skelettalen Klassen, das heißt insbesondere ein Bestimmen, ob ein Vorbiss oder eine Überbiss vorliegt. Zur Ermittlung der skelettalen Klasse ist es bevorzugt zusätzlich zu den mesial gelegenen Punkten des retromolaren Dreiecks die tiefsten Punkte des Mundhöhlenvorhofs auf der rechten und linken Seite neben dem Lippenbändchen des Unterkiefers zu bestimmen und als zusätzlich Basispunkte zu definieren. Insbesondere mit Hilfe dieser Basispunkte erfolgt ein Bestimmen eines Bisswinkels $k_c$ und eine Bisshöhe h, die sodann zur Ermittlung der skelettalen Klasse genutzt wird.

Hierbei kann zur Ermittlung des Bisswinkels $k_c$ zunächst eine Mittellinie zwischen den beiden definierten mesial gelegenen Punkten und den beiden tiefsten Punkten des Mundhöhlenvorhofs bestimmt werden. Die zweite Linie wird sodann durch eine Verbindungslinie zwischen dem Mittelpunkt der beiden tiefsten Punkte der Mundhöhlenvorhöfe und einem Punkt am Oberkiefer, dem Punkt Papilla Incisiva definiert.

Der Absatz zwischen dem mittleren Punkt zwischen den beiden tiefsten Punkten der Mundhöhlenvorhöfe und dem Punkt Papilla Incisiva definiert ferner die Bisshöhe h.

Zur mathematischen Bestimmung werden die Koordinaten der beiden mesialen Punkte rdr und rdl sowie der beiden tiefsten Punkte des Mundhöhlenvorhofs mvr und mvl bestimmt zu:

$$rdr\ (x_{rdr};\ y_{rdr};\ z_{rdr})$$
$$rdl\ (x_{rdl};\ y_{rdl};\ z_{rdl})$$
$$mvr\ (x_{mvr};\ y_{mvr}; z_{mvr})$$
$$mvl\ (x_{mvl};\ y_{mvl};\ z_{mvl}) \quad (Gl.\ 3)$$

wobei x, y, z die Koordinaten und r rechts sowie l links bezeichnet.

Die gesuchten Mittelpunkte

$$rdm\ (x_{rdm};\ y_{rdm},\ z_{rdm})$$
$$mvm\ (y_{mvm};\ y_{mvm};\ z_{mvm}) \quad (Gl.\ 4)$$

berechnen sich aus

$$rdm \left( \frac{x_{rdl} + x_{rdr}}{2} ; \frac{y_{rdl} + y_{rdr}}{2} ; \frac{z_{rdl} + z_{rdr}}{2} \right)$$

$$mvm \left( \frac{x_{mvl} + x_{mvr}}{2} ; \frac{y_{mvl} + y_{mvr}}{2} ; \frac{z_{mvl} + z_{mvr}}{2} \right) \quad (Gl.\ 5)$$

Ferner wurden die Koordinaten des Punktes Papilla Incisiva pi ($x_{pi}$; $y_{pi}$; $z_{pi}$) bestimmt. Hieraus kann die Bisshöhe h sowie der Bisswinkel $k_c$ berechnet werden durch

$$h\ (mvm;\ pi) = \sqrt{(x_{mvm} - x_{pi})^2 + (y_{mvm} - y_{pi})^2 + (z_{mvm} - z_{pi})^2}$$

$$a\ (rdm;\ pi) = \sqrt{(x_{rdm} - x_{pi})^2 + (y_{rdm} - y_{pi})^2 + (z_{rdm} - z_{pi})^2}$$

$$b\ (rdm;\ mvm) = \sqrt{(x_{rdm} - x_{mvm})^2 + (y_{rdm} - y_{mvm})^2 + (z_{rvm} - z_{mvm})^2}$$

$$kc = \arccos\left(\frac{a^2 - b^2 - h^2}{-2bh}\right) \tag{Gl. 6}$$

Auf Basis der erhaltenen Werte für den Bisswinkel kc und die Bisshöhe h kann automatisch eine skelettale Klasse festgelegt werden. Dies erfolgt insbesondere durch hinterlegte Grenzwerte. Beispielsweise liegt von der skelettalen Klasse vor:

$$\text{Normalbiss (Klasse1): } kc > 50° \text{ und } \leq 90°$$

$$\text{Überbiss (Klasse 2): } kc > 90°$$

$$\text{Vorbiss (Klasse 3): } kc \leq 50° \tag{Gl. 7}$$

Selbstverständlich ist es auch möglich, Zwischenklassen zu definieren, um eine weitere Verfeinerung des erfindungsgemäßen Verfahrens zu realisieren.

Zur automatischen Bestimmung der Bissart werden zwei interalveoläre Verbindungswinkel $\alpha$ beziehungsweise $\beta$ bestimmt. Der auf der linken Seite des Unterkiefers zu bestimmende Winkel $\alpha$ kann berechnet werden aus

$$w\ (rdl;\ tl) = \sqrt{(x_{rdl} - x_{tl})^2 + (y_{rdl} - y_{tl})^2 + (z_{rdl} - z_{tl})^2}$$

$$q\ (tl;\ rdr) = \sqrt{(x_{tl} - x_{rdr})^2 + (y_{tl} - y_{rdr})^2 + (z_{tl} - z_{rdr})^2}$$

$$dr\ (rdr;\ rdl) = \sqrt{(x_{rdr} - x_{rdl})^2 + (y_{rdr} - y_{rdl})^2 + (z_{rdr} - z_{rdl})^2}$$

$$\alpha = \arccos\left(\frac{q^2 - w^2 - dr^2}{-2wdr}\right) \tag{Gl. 8}$$

wobei

w der Abstand zwischen dem linken am weitesten mesial gelegenen Punkt und dem linken Punkt Tuber Maxillae ist.

Der Winkel $\beta$ auf der rechten Seite kann bestimmt werden durch

$$v\ (rdr;\ tr) = \sqrt{(x_{rdr} - x_{tr})^2 + (y_{rdr} - y_{tr})^2 + (z_{rdr} - z_{tr})^2}$$

$$p\ (tr;\ rdl) = \sqrt{(x_{tr} - x_{rdl})^2 + (y_{tr} - y_{rdl})^2 + (z_{tr} - z_{rdl})^2}$$

$$dr\ (rdr;\ rdl) = \sqrt{(x_{rdr} - x_{rdl})^2 + (y_{rdr} - y_{rdl})^2 + (z_{rdr} - z_{rdl})^2}$$

$$\beta = \arccos\left(\frac{q^2 - v^2 - dr^2}{-2vdr}\right) \tag{Gl. 9}$$

wobei

v der Abstand zwischen dem rechten am weitesten mesial gelegenen Punkt und dem rechten Punkt Tuber Maxillae ist.

Als Bissart ergibt sich aus den Berechnungen sodann

$$\text{Normalbiss: } \alpha \text{ und } \beta \geq 80°$$

$$\text{Beidseitiger Kreuzbiss: } \alpha \text{ und } \beta < 80°$$

$$\text{Einseitiger Kreuzbiss: } \alpha \text{ oder } \beta < 80° \tag{Gl. 10}$$

In besonderes bevorzugter Ausführungsform der Erfindung erfolgt somit auf Basis des ermittelten Transversalmaßes, der ermittelten skelettalen Klasse und der ermittelten Bissart durch das System ein Vorschlag einer Auswahl für eine passende Zahnstellung. Aus einer großen Anzahl von Zahnaufstellungen, die 10 bis 20 Zahnaufstellungen umfassen kann, wird dem Zahntechniker oder Zahnarzt mit Hilfe des erfindungsgemäßen Verfahrens eine Auswahl von maximal 2 bis 5 Aufstellungen angeboten, die sich maßgeblich durch ästhetische Formabweichungen in der Front unterscheiden.

Eine derart geringe Anzahl von möglichen Aufstellungen erfolgt einerseits durch die automatische Berechnung des Transversalmaßes, der skelettalen Klasse und der Bissart, sowie insbesondere auch aufgrund manueller Auswahlen. Insbesondere erfolgt eine manuelle Auswahl eines Okklusionskonzepts und/oder einer Zahnlinie und/oder einer Zahnform.

Bei dem Okklusionskonzept kann der Anwender vorzugsweise aus mehreren Okklusionskonzepten auswählen. Hierbei handelt es sich insbesondere um eine Auswahl nach:

- Aufstellung über Bukkalkontakte
- Aufstellung lingualisiert
- Aufstellung nach Prof. Dr. Dr. Grunert
- Aufstellung nach Prof. Dr. Gerber

In bevorzugter Ausführungsform werden dem Benutzer für die Auswahl der Zahnlinie ebenfalls unterschiedliche Zahnlinienkombinationen angeboten.

Hierbei handelt es sich beispielsweise um:

- VITAPAN PLUS® (Frontzähne) und VITA LINGOFORM® (Seitenzähne)
- VITAPAN EXCELL® (Frontzähne) und VITA LINGOFORM® (Seitenzähne)
- - VITA VIONIC® (Front- und Seitenzähne)

Vorzugsweise wird dem Benutzer auch bei der Zahnform eine Auswahl angeboten. Hierbei handelt es sich beispielsweise um:

- Form R (Rechteckig)
- Form O (Schaufelförmig)
- Form T (Dreieckig)

Bei einer weiteren bevorzugten Ausführungsform der Erfindung, durch die die Anzahl und/oder die Qualität der nach der Berechnung angebotenen Aufstellung verringert bzw. verbessert werden kann, erfolgt das Auswählen einer Frontzahngarnitur auf Basis der Nasenbreite. Hierbei ist es ausreichend die insbesondere ausreichend die Nasenbreite ebenfalls in das System einzugeben. Hierbei wird die Nasenbreite gegebenenfalls mit einem Faktor multipliziert. Der Faktor liegt vorzugsweise im Bereich von ca. +/- 2mm

Nachstehend wird die Erfindung anhand der anliegenden Zeichnungen bezüglich einer bevorzugten Ausführungsform näher erläutert.

Es zeigen:

Fig. 1        ein schematisches Ablaufdiagramm für die Auswahl einer bestmöglichen Aufstellung für den Patienten unter Zuhilfenahme des erfindungsgemäßen Verfahrens,

Fig. 2        eine Übersicht der Filterkriterien,

Fig. 3        eine schematische Ansicht eines digitalisierten Unterkiefers mit den zu bestimmenden Basispunkten,

Fig. 4        eine schematische Ansicht eines digitalisierten Oberkiefer mit den zu bestimmenden Basispunkten,

Fig. 5        eine schematische Ansicht des digitalen Unterkiefers zur Bestimmung des Transversalmaßes,

Fig. 6        eine Tabelle sowie eine Skizze zur Ermittlung des Transversalmaßes,

Fig. 7        eine schematische digitale Ansicht des Unterkiefers zur Ermittlung der skelettalen Klasse,

Fig. 8        eine schematische Seitenansicht des digitalisierten Unter- und Oberkiefers zur Ermittlung der Bisshöhe h und des Bisswinkels $k_c$,

Fig. 9          eine schematische Rückansicht des digitalisierten Ober- und Unterkiefers zur Ermittlung der Bissart,

Fig. 10          eine schematische Ansicht zur Ermittlung des Garniturmaßes und

Fig. 11 und 12          Beispiele von mit dem erfindungsgemäßen Verfahren bestimmten Aufstellungen.

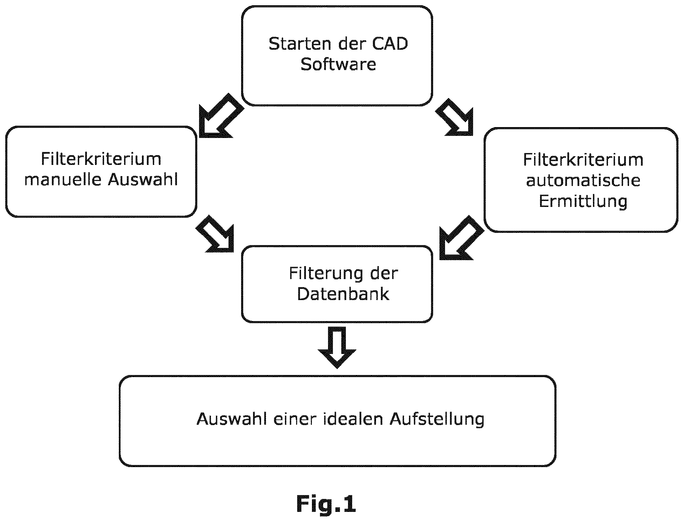

In Fig. 1 ist schematisch der wesentliche Ablauf zur Auswahl einer idealen Zahnaufstellung dargestellt. In einem ersten Schritt wird die Software, bei der es sich insbesondere um eine CAD Software handelt gestartet. Hierbei erfolgt insbesondere auch das Einlesen der digitalisierten oralen Patientensituation, wobei dies selbstverständlich auch zu einem früheren Zeitpunkt bereits erfolgt sein kann. In den nächsten Schritten erfolgt einerseits eine manuelle Auswahl von Filterkriterien und andererseits eine automatische Ermittlung von Filterkriterien auf Grundlage von Berechnungen. Sämtliche Filterkriterien werden sodann im nächsten Schritt zur Filterung der Datenbank, in der die Vielzahl von Zahnaufstellungen hinterlegt ist, genutzt. Hieraus ergibt sich eine Auswahl von nur wenigen geeigneten Zahnaufstellungen, aus denen sodann insbesondere der Zahntechniker auswählen kann. Bei den Zahnaufstellungen handelt es sich um bereits zu einer (Teil-)Zahnreihe zusammengefügte künstliche Zähne, welche zueinander optimal angepasst sind. Einzelzähne müssen somit nicht mehr zusammengefügt werden, sondern sind bereits in der Zahnaufstellung enthalten.

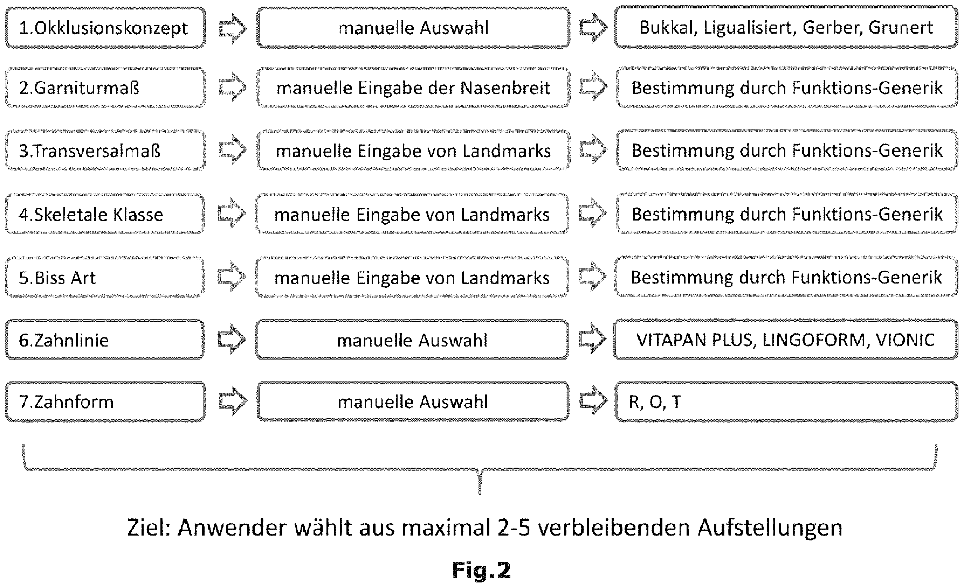

Fig. 2 zeigt eine Übersicht der Filterkriterien. Hierbei sind die mit 1, 6 und 7 bezeichneten Filterkriterien manuell auszuwählende Filterkriterien. Hierbei handelt es sich um das Okklusionskonzept, die Zahnlinie und die Zahnform, wobei auf der rechten Seite der Fig. 2 Beispiele möglicher, für die Benutzer angebotener Auswahlen angegeben sind.

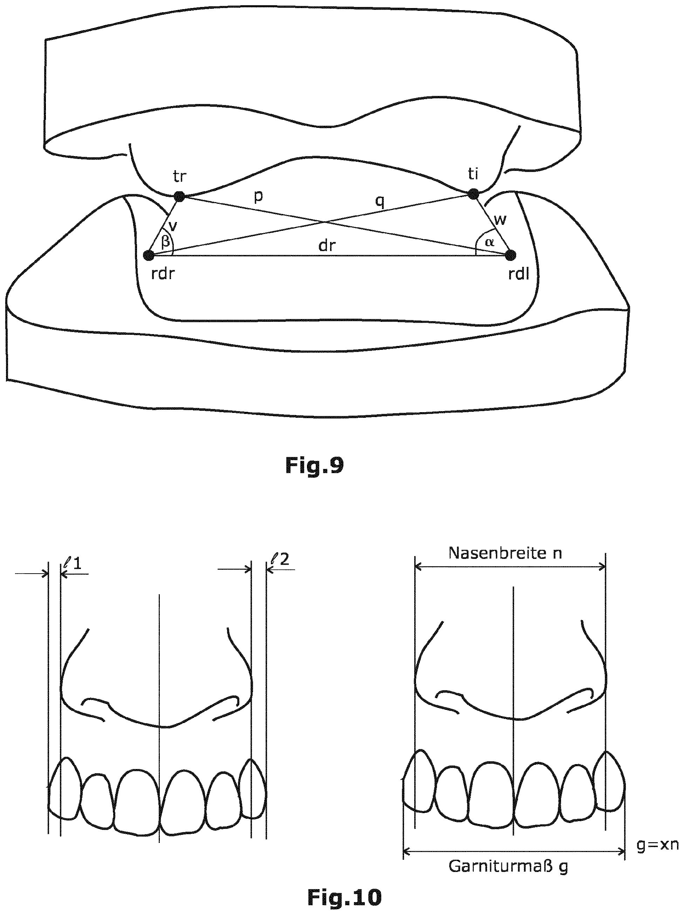

Für die Bestimmung des Garniturmaßes erfolgt vom Benutzer eine manuelle Eingabe der Nasenbreite, wie später an Fig. 10 erläutert.

Zur Bestimmung des Transversalmaßes, der skelektalen Klasse und der Bissart werden unterschiedliche Basispunkte bzw. Landmarks manuell eingegeben oder vom System automatisch ermittelt. Mit Hilfe der hinterlegten Algorithmen werden die entsprechenden Kriterien automatisch bestimmt.

Die Kombination sämtlicher Filterkriterien erfolgt zur Auswahl der idealen Aufstellung anhand der in einer Datenbank hinterlegten großen Anzahl unterschiedlicher Aufstellungen.

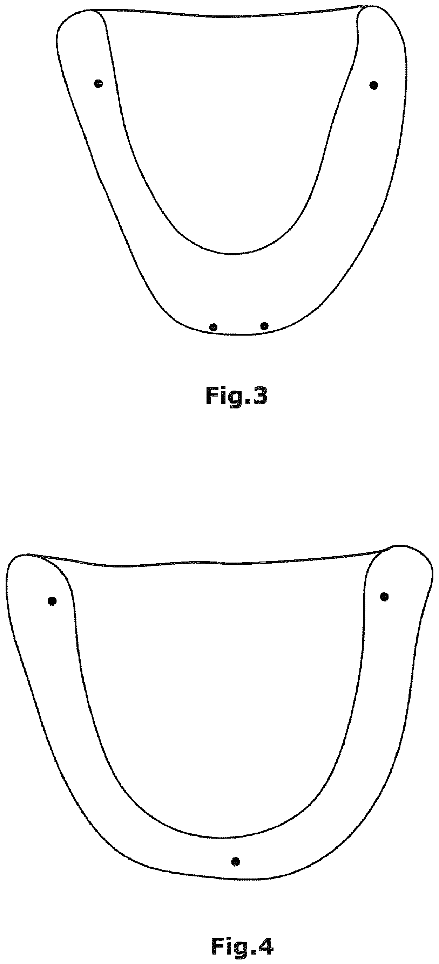

Für die einzelnen durchzuführenden Berechnungen werden Basispunkte bzw. Landmarks definiert. Hierzu wird der Unterkiefer wie in Fig. 3 dargestellt digitalisiert im Rechner abgebildet. Als Basispunkte werden die beiden mesial gelegenen Punkte rdl und rdr auf der linken (l) bzw. auf der rechten Seite (r) des Unterkiefers definiert.

Ferner werden die beiden tiefsten Punkte mvr und mvl des Mundhöhlenvorhofs auf der rechten und linken Seite neben dem Lippenbändchen definiert.

Da der Oberkiefer ebenfalls in digitalisierter Form in dem Rechner abgebildet ist (Fig. 4), erfolgt ferner die Definition des linken und rechten Punktes Tuber Maxillae (tr, tl). Ferner wird im Wesentlichen auf der dem Lippenbändchen des Unterkiefers gegenüberliegenden Seite des Oberkiefers der Punkt Papilla Incisiva (pi) ermittelt.

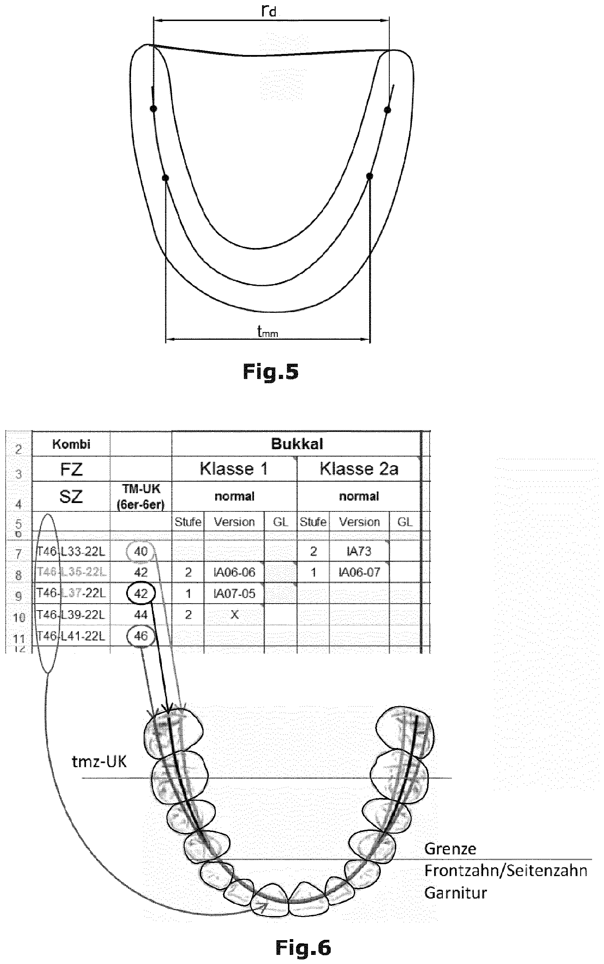

Zur Ermittlung des Transversalmaßes wird, wie in Fig. 5 dargestellt, der Abstand $d_r$ ermittelt. Bei diesem handelt es sich um den Abstand zwischen den beiden mesialen Punkten rdr und rdl. Der Abstand $r_d$ wird sodann mit einem Faktor y multipliziert um gemäß Gleichung 1 das Transversalmaß $t_{mm}$ zu erhalten.

Auf Basis des ermittelten Transversalmaßes kann, wie in Fig. 6 beispielhaft dargestellt eine Aufstellung ermittelt werden. Hierbei können Aufstellungen mit gleicher Frontzahngröße unterschiedliche Zahnbogenmaße aufweisen. Im dargestellten Beispiel wird über das Filterkriterium das Transversalmaß der Aufstellung $t_{ma}$, Mittelfissur 36:46 genutzt, um eine passende Aufstellung auszuwählen. $T_{ma}$ muss hierbei innerhalb des ermittelten Transversalmaßes $T_{mm} \pm$ 1,5mm liegen.

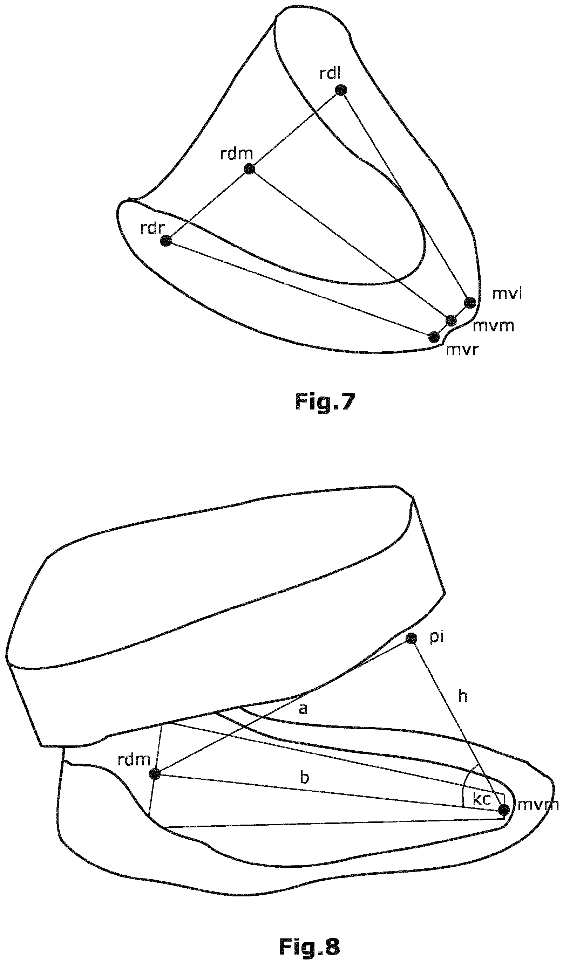

Zur Ermittlung der skelettalen Klasse werden zunächst wie in Fig. 7 dargestellt die beiden definierten mesialen Punkte rdl und rdr miteinander verbunden und der Mittelpunkt rdm zwischen diesen beiden Punkten bestimmt. Entsprechend werden die beiden Punkte im Mundvorhof mvr und mvl miteinander verbunden und wiederum deren Mittelpunkt mvm bestimmt. Diese ergibt eine Verbindungslinie b. Zur Definition einer Verbindunglinie h (Fig. 8) erfolgt ein Verbinden des Punktes mvm mit dem am Oberkiefer definierten Punkt pi (Fig. 4) hieraus ergibt sich der Winkel $k_c$. Des Weiteren wird eine Verbindungslinie a) zwischen den Punkten rdm und pi definiert. Die Berechnung des Winkels erfolgt über die Gleichung 6.

Die Berechnung der Bissart erfolgt anhand der Gleichung 8 und anhand der Gleichung 9 sowie anhand der in Fig. 9 dargestellten Geometrie. Zusätzlich zu dem Abstand $d_r$ zwischen den Punkten rdr und rdl werden die Strecken v zwischen den Punkten rdr und tr sowie w zwischen den Punkten rdl und tl definiert. Des Weiteren erfolgt eine Definition der Strecken p zwischen den Punkten rdl und tr sowie q zwischen den Punkten rdr und tl. Aus den Gleichungen 7 und 8 können sodann die Winkel $\alpha$ und $\beta$ bestimmt werden. Hieraus ergibt sich sodann die Bissart.

Zur Bestimmung der Frontzahngarnitur erfolgt ein Messen der Nasenbreite n (Fig. 10). Zur Nasenbreite n wird l1 + l2 addiert, wobei l1, l2 üblicherweise im Bereich von 0,5 - 3,5 mm liegen. l1 und l2 entsprechen dem Abstand

zwischen einer Außenseite des Nasenflügels und einer Außenseite des Eckzahns wie in Fig. 10 dargestellt. Hieraus ergibt sich das Garniturmaß g entsprechend der Gleichung

$$g = l1 + l2 + n \qquad (Gl. 11)$$

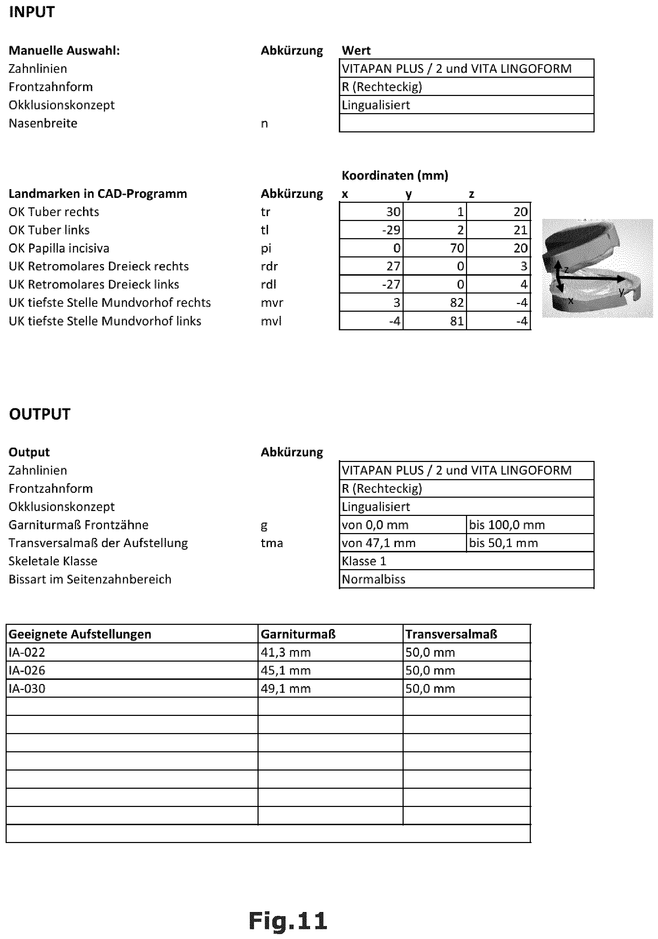

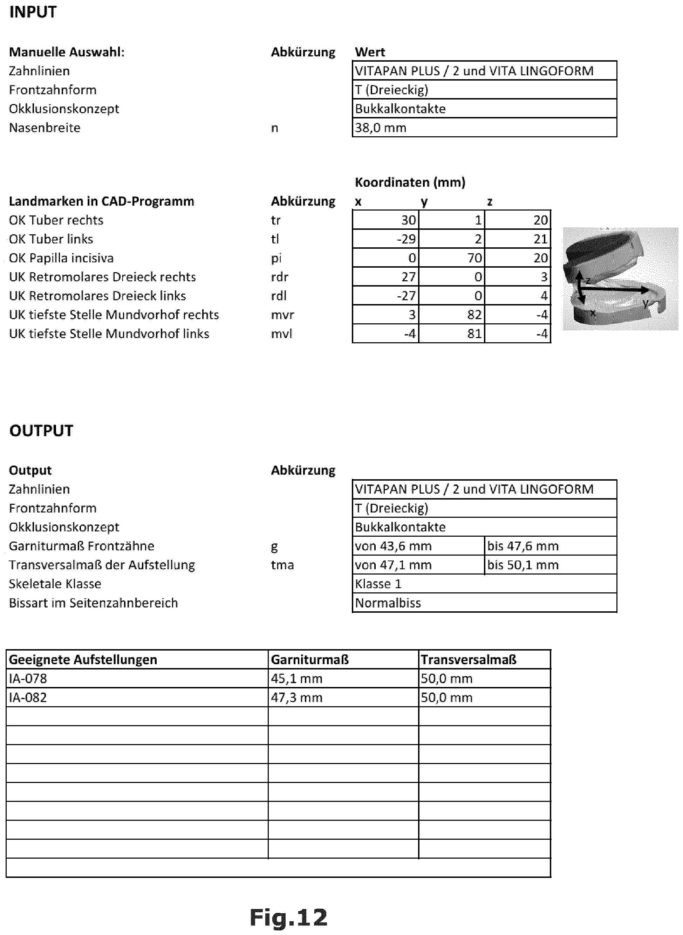

Wie in den Fign. 11 und 12 sind Beispiele für Berechnungen dargestellt. Als manuelle Auswahl bzw. Eingabe erfolgt das Eingeben der Zahnlinie, der Frontzahnform, des Okklusionskonzepts und der Nasenbreite.

Beispiel: Nasenbreite 40mm (Eckzahnmitte zu Eckzahnmitte), Zahnform gemäß Typ O

g = 3,0 + 3,0 + 40 [mm]
g = 46,0, was ungefähr O45 entspricht

Aus den in das CAD Programm eingelesenen bzw. digitalisierten oralen Patientensituationen ergeben sich die Landmarken bzw. Basispunkte mit den entsprechenden Koordinaten. Mit Hilfe erfindungsgemäßen Verfahrens erfolgt sodann ein Filtern von gemäß dem Beispiel in Fig. 11 drei und dem Beispiel in Fig. 12 zwei geeigneten Aufstellungen aus einer Vielzahl in einer Datenbank hinterlegten Aufstellungen. Der Zahntechniker braucht sodann lediglich aus den ermittelten passenden Zahnaufstellungen zu wählen. Fehlerquellen bei der Erstellung der Zahn(teil)prothese beispielsweise durch fehlerhaft zueinander angepasste Einzelzähne entfallen vollständig. Dem Zahntechniker wird ein optimaler Ausgangspunkt zur Erstellung der Zahn(teil)prothese gegeben, ohne dass es hierbei auf die Erfahrung bzw. handwerkliche Geschicklichkeit des Zahntechnikers ankommt.

**Patentansprüche**

1. Computerimplementiertes Verfahren zur Festlegung einer Zahnaufstellung für die Herstellung von Zahnteil- oder Vollprothese mit den Schritten:

   Bestimmen von durch Koordinaten definierte Basispunkte eines Unterkiefers und/oder einer Oberkiefers;
   Ermitteln eines Transversalmaßes und/oder einer skelettalen Klasse und/oder einer Bissart auf Basis der bestimmten Koordinaten für den Unterkiefer und/oder den Oberkiefer und
   Auswahl, aus einer Vielzahl von vorherbestimmten Zahnaufstellungen, einer passenden Zahnaufstellung, welche zueinander passende künstliche Zähne enthält auf Grundlage des ermittelten Transversalmaßes und/oder der ermittelten skelettalen Klasse und/oder der ermittelten Bissart.

2. Computerimplementiertes Verfahren nach Anspruch 1, **dadurch gekennzeichnet, dass** die Basispunkte, die am weitesten mesial gelegenen Punkte (rdl, rdr) der retromolaren Dreiecke auf der rechten und linken Seite des Unterkiefers umfassen.

3. Computerimplementiertes Verfahren nach Anspruch 1 oder 2, **dadurch gekennzeichnet, dass** die Basispunkte, die tiefsten Punkte (mvl, mvr) des Mundhöhlenvorhofs auf der rechten und linken Seite neben dem Lippenbändchen der Unterkieferbasis umfassen.

4. Computerimplementiertes Verfahren nach einem der Ansprüche 1 bis 3, **dadurch gekennzeichnet, dass** die Basispunkte die Punkte (tl, tr) Tuber Maxillae auf der rechten und linken Seite des Oberkiefers umfassen.

5. Computerimplementiertes Verfahren nach einem der Ansprüche 1 bis 4, **dadurch gekennzeichnet, dass** die Basispunkte den Punkt (pi) Papilla Incisiva des Oberkiefers umfassen.

6. Computerimplementiertes Verfahren nach einem der Ansprüche 2 bis 5, **dadurch gekennzeichnet, dass** das Transversalmaß ($t_{mm}$) zumindest auf Basis des Abstandes ($r_d$) der mesialen Punkte (tl, tr) des retromolaren Dreiecks ermittelt wird.

7. Computerimplementiertes Verfahren nach Anspruch 6, **dadurch gekennzeichnet, dass** das Transversalmaß mit einem Korrekturfaktor (y) multipliziert wird.

8. Computerimplementiertes Verfahren nach einem der Ansprüche 1 bis 7, **dadurch gekennzeichnet, dass** eine skelettale Klasse auf Basis eines Bisswinkels ($k_c$) und einer Bisshöhe (h) ermittelt wird.

9. Computerimplementiertes Verfahren nach Anspruch 8, bei welchem der Bisswinkel ($k_c$) zwischen einer Mittellinie (b), die in der Mitte (mdm, mvm) zwischen den am weitesten mesial gelegenen Punkten (tdl, tdr) und den tiefsten Punkten (mvl, mvr) der Mundhöhlenvorhöfe verläuft und einer Verbindungslinie (h) zu dem Punkt (pi) Papilla Incisiva verläuft.

10. Computerimplementiertes Verfahren nach Anspruch 8 oder 9, wobei die Bisshöhe (h) bestimmt wird als Abstand zwischen dem Mittelpunkt mvm der beiden tiefsten Punkte (mvl, mvr) im Mundvorhof und dem Punkt (pi) Papilla Incisiva.

11. Computerimplementiertes Verfahren nach einem der Ansprüche 1 bis 10, bei welchem die Bissart auf Basis der interalveolären Verbindungswinkel ($\alpha$, $\beta$) bestimmt wird.

12. Computerimplementiertes Verfahren nach Anspruch 11, bei welchem die Verbindungswinkel ($\alpha$, $\beta$) bestimmt werden durch die Verbindungslinie (v, w, dr) zwischen den beiden am weitesten mesial gelegenen Punkten (tr, tl) des retromolaren Dreiecks und dem linken bzw. rechten Punkt (tr, tl) Tubera.

13. Computerimplementiertes Verfahren nach Anspruch 11 oder 12, bei welchem ein Normalbiss bestimmt wird, wenn die beiden Verbindungswinkel $\leq 80°$ sind, bei welchem ein beidseitiger Kreuzbiss bestimmt wird, wenn die beiden Verbindungswinkel $\geq 80°$ sind und bei welchem einer einseitiger Kreuzbiss bestimmt wird, wenn nur einer der beiden Verbindungswinkel $\geq 80°$ ist.

14. Computerimplementiertes Verfahren nach einem der Ansprüche 1 bis 13, bei welchem eine Frontzahngarnitur auf Basis einer Nasenbreite n ausgewählt wird, wobei die Nasenbreite (n) gegebenenfalls mit einem Korrekturfaktor (x) multipliziert wird.

15. Computerimplementiertes Verfahren nach einem der Ansprüche 1 bis 14, bei welchem zur Auswahl einer Zahnaufstellung für die Unterkieferbasis und/oder die Oberkieferbasis ein Okklusionskonzept und/oder eine Zahnlinie und/oder eine Zahnform ausgewählt werden.

**Claims**

1. Computer-implemented method for defining a dental arrangement for the production of partial or full tooth prostheses, comprising the steps of:

   determining base points of a lower jaw and/or an upper jaw, defined by coordinates;
   determining a transverse dimension and/or a skeletal class and/or type of bite on the basis of the coordinates determined for the lower jaw and/or the upper jaw, and
   selecting, from a plurality of predetermined dental arrangements, a suitable dental arrangement on the basis of the transverse dimension determined and/or the skeletal class determined and/or the type of bite determined.

2. Computer-implemented method of claim 1, **characterized in that** the base points include the farthest mesial points (rdl, rdr) of the retromolar triangles on the right and the left sides of the lower jaw.

3. Computer-implemented method of claim 1 or 2, **characterized in that** the base points include the lowest points (mvl, mvr) of the oral vestibule on the right and left sides beside the labial frenulum of the lower jaw.

4. Computer-implemented method of one of claims 1 to 3, **characterized in that** the base points include the points (tl, tr) tuber maxillae on the right and left sides of the upper jaw.

5. Computer-implemented method of one of claims 1 to 4, **characterized in that** the base points include the point (pi) papilla incisiva of the upper jaw.

6. Computer-implemented method of one of claims 2 to 5, **characterized in that** the transverse dimension ($t_{mm}$) is determined at least on the basis of the distance ($r_d$) of the mesial points (tl, tr) of the retromolar triangle.

7. Computer-implemented method of claim 6, **characterized in that** the transverse dimension is multiplied by a correction factor (y).

8. Computer-implemented method of one of claims 1 to 7, **characterized in that** a skeletal class is determined on the basis of an occlusal angle ($k_c$) and an occlusal height (h).

9. Computer-implemented method of claim 8, wherein the occlusal angle ($k_c$) extends between a centerline (b) which extends in the middle (mdm, mvm) between the farthest mesial points (tdl, tdr) and the lowest points (mvl, mvr) of the oral vestibule, and a connecting line (h) to the point (pi) papilla incisiva.

10. Computer-implemented method of claim 8 or 9, wherein the occlusal height (h) is determined as the distance between the center (mvm) of the two lowest points (mvl, mvr) in the oral vestibule and the point (pi) papilla incisiva.

11. Computer-implemented method of one of claims 1 to 10, wherein the type of bite is determined based on the interalveolar connecting angles ($\alpha$, $\beta$).

12. Computer-implemented method of claim 11, wherein the connecting angles ($\alpha$, $\beta$) are determined by the connecting line (v, w, dr) between the two farthest mesial points (tr, tl) of the retromolar triangle and the left or right point (tr, tl) tubera, respectively.

13. Computer-implemented method of claim 11 or 12, wherein a normal occlusion is determined, if the two connecting angles are $\leq 80°$, wherein a bilateral crossbite is determined, if the two connecting angles are $\geq 80°$, and wherein a unilateral crossbite is determined, if only one of the two connecting angles is $\geq 80°$.

14. Computer-implemented method of one of claims 1 to 13, wherein a set of anterior teeth is selected based on a nose width (n), wherein the nose width (n) is multiplied by a correction factor (x), if applicable.

15. Computer-implemented method of one of claims 1 to 14, wherein for the selection of a tooth arrangement for the lower jaw base and/or the upper jaw base, an occlusal concept and/or a tooth line and/or a tooth shape are selected.

**Revendications**

1. Procédé mis en œuvre par ordinateur et destiné à identifier une configuration dentaire pour la production de prothèses dentaires partielles ou complètes, comprenant les étapes ci-dessous consistant à :

   déterminer des points de base, définis grâce à des coordonnées, d'une mandibule et/ou d'un maxillaire supérieur ;
   déterminer une mesure transversale et/ou une classe squelettique et/ou un type d'occlusion en se basant sur les coordonnées déterminées pour la mandibule et/ou le maxillaire supérieur et
   sélectionner, parmi une pluralité de configurations dentaires prédéfinies, une configuration dentaire adaptée contenant des dents artificielles compatibles entre elles sur la base de la mesure transversale déterminée et/ou de la classe squelettique déterminée et/ou du type d'occlusion déterminé.

2. Procédé mis en œuvre par ordinateur selon la revendication 1, **caractérisé en ce que** les points de base comprennent les points les plus mésiaux (rdl, rdr) des triangles rétromolaires sur les côtés droit et gauche de la mandibule.

3. Procédé mis en œuvre par ordinateur selon la revendication 1 ou 2, **caractérisé en ce que** les points de base comprennent les points les plus profonds (mvl, mvr) du vestibule buccal sur les côtés droit et gauche à proximité du frein labial de la base de mandibule.

4. Procédé mis en œuvre par ordinateur selon l'une des revendications 1 à 3, **caractérisé en ce que** les points de base comprennent les points (tl, tr) des tubérosités maxillaires sur les côtés droit et gauche du maxillaire supérieur.

5. Procédé mis en œuvre par ordinateur selon l'une des revendications 1 à 4, **caractérisé en ce que** les points de base comprennent le point (pi) de la papille incisive du maxillaire supérieur.

6. Procédé mis en œuvre par ordinateur selon l'une des revendications 2 à 5, **caractérisé en ce que** la mesure

transversale ($t_{mm}$) est déterminée au moins en se basant sur l'espacement ($r_d$) entre les points mésiaux (tl, tr) du triangle rétromolaire.

7. Procédé mis en œuvre par ordinateur selon la revendication 6, **caractérisé en ce que** la mesure transversale est multipliée par un facteur de correction (y).

8. Procédé mis en œuvre par ordinateur selon l'une des revendications 1 à 7, **caractérisé en ce qu'**une classe squelettique est déterminée en se basant sur un angle d'occlusion ($k_c$) et sur une hauteur d'occlusion (h).

9. Procédé mis en œuvre par ordinateur selon la revendication 8, dans lequel l'angle d'occlusion ($k_c$) s'inscrit entre une ligne médiane (b), passant au milieu (mdm, mvm) entre les points les plus mésiaux (tdl, tdr) et les points les plus profonds (mvl, mvr) des vestibules buccaux, et une ligne de liaison (h) allant jusqu'au point (pi) de la papille incisive.

10. Procédé mis en œuvre par ordinateur selon la revendication 8 ou 9, dans lequel la hauteur d'occlusion (h) est définie comme étant l'espacement entre le point médian mvm des deux points les plus profonds (mvl, mvr) du vestibule buccal et le point (pi) de la papille incisive.

11. Procédé mis en œuvre par ordinateur selon l'une des revendications 1 à 10, dans lequel le type d'occlusion est déterminé en se basant sur les angles de liaison interalvéolaires ($\alpha$, $\beta$).

12. Procédé mis en œuvre par ordinateur selon la revendication 11, dans lequel les angles de liaison ($\alpha$, $\beta$) sont déterminés par la ligne de liaison (v, w, dr) s'étendant entre les deux points les plus mésiaux (tr, tl) du triangle rétromolaire et le point gauche ou droit (tr, tl) des tubérosités maxillaires.

13. Procédé mis en œuvre par ordinateur selon la revendication 11 ou 12, dans lequel une occlusion normale est déterminée lorsque les deux angles de liaison sont $\leq 80°$, dans lequel une occlusion croisée bilatérale est déterminée lorsque les deux angles de liaison sont $\geq 80°$ et dans lequel une occlusion croisée unilatérale est déterminée lorsqu'un seul des deux angles de liaison est $\geq 80°$.

14. Procédé mis en œuvre par ordinateur selon l'une des revendications 1 à 13, dans lequel un jeu de dents de devant est sélectionné en se basant sur une largeur de nez n, la largeur de nez (n) étant éventuellement multipliée par un facteur de correction (x).

15. Procédé mis en œuvre par ordinateur selon l'une des revendications 1 à 14, dans lequel un concept d'occlusion et/ou un alignement dentaire et/ou une forme dentaire sont sélectionnés en vue de la sélection d'une configuration dentaire pour la base de mandibule et/ou la base de maxillaire supérieur.

Starten der CAD
Software

Filterkriterium
manuelle Auswahl

Filterkriterium
automatische
Ermittlung

Filterung der
Datenbank

Auswahl einer idealen Aufstellung

**Fig.1**

| | | |
|---|---|---|
| 1.Okklusionskonzept | → manuelle Auswahl | → Bukkal, Ligualisiert, Gerber, Grunert |
| 2.Garniturmaß | → manuelle Eingabe der Nasenbreit | → Bestimmung durch Funktions-Generik |
| 3.Transversalmaß | → manuelle Eingabe von Landmarks | → Bestimmung durch Funktions-Generik |
| 4.Skeletale Klasse | → manuelle Eingabe von Landmarks | → Bestimmung durch Funktions-Generik |
| 5.Biss Art | → manuelle Eingabe von Landmarks | → Bestimmung durch Funktions-Generik |
| 6.Zahnlinie | → manuelle Auswahl | → VITAPAN PLUS, LINGOFORM, VIONIC |
| 7.Zahnform | → manuelle Auswahl | → R, O, T |

Ziel: Anwender wählt aus maximal 2-5 verbleibenden Aufstellungen

**Fig.2**

EP 3 454 776 B1

Fig.3

Fig.4

**Fig.5**

| Kombi | | Bukkal | | | | | |
|---|---|---|---|---|---|---|---|
| FZ | | Klasse 1 | | | Klasse 2a | | |
| SZ | TM-UK (6er-6er) | normal | | | normal | | |
| | | Stufe | Version | GL | Stufe | Version | GL |
| T46-L33-22L | 40 | | | | 2 | IA73 | |
| T46-L35-22L | 42 | 2 | IA06-06 | | 1 | IA06-07 | |
| T46-L37-22L | 42 | 1 | IA07-05 | | | | |
| T46-L39-22L | 44 | 2 | X | | | | |
| T46-L41-22L | 46 | | | | | | |

tmz-UK

Grenze
Frontzahn/Seitenzahn
Garnitur

**Fig.6**

**Fig.7**

**Fig.8**

**Fig.9**

**Fig.10**

## INPUT

| Manuelle Auswahl: | Abkürzung | Wert |
|---|---|---|
| Zahnlinien | | VITAPAN PLUS / 2 und VITA LINGOFORM |
| Frontzahnform | | R (Rechteckig) |
| Okklusionskonzept | | Lingualisiert |
| Nasenbreite | n | |

| Landmarken in CAD-Programm | Abkürzung | x | y | z |
|---|---|---|---|---|
| | | Koordinaten (mm) | | |
| OK Tuber rechts | tr | 30 | 1 | 20 |
| OK Tuber links | tl | -29 | 2 | 21 |
| OK Papilla incisiva | pi | 0 | 70 | 20 |
| UK Retromolares Dreieck rechts | rdr | 27 | 0 | 3 |
| UK Retromolares Dreieck links | rdl | -27 | 0 | 4 |
| UK tiefste Stelle Mundvorhof rechts | mvr | 3 | 82 | -4 |
| UK tiefste Stelle Mundvorhof links | mvl | -4 | 81 | -4 |

## OUTPUT

| Output | Abkürzung | | |
|---|---|---|---|
| Zahnlinien | | VITAPAN PLUS / 2 und VITA LINGOFORM | |
| Frontzahnform | | R (Rechteckig) | |
| Okklusionskonzept | | Lingualisiert | |
| Garniturmaß Frontzähne | g | von 0,0 mm | bis 100,0 mm |
| Transversalmaß der Aufstellung | tma | von 47,1 mm | bis 50,1 mm |
| Skeletale Klasse | | Klasse 1 | |
| Bissart im Seitenzahnbereich | | Normalbiss | |

| Geeignete Aufstellungen | Garniturmaß | Transversalmaß |
|---|---|---|
| IA-022 | 41,3 mm | 50,0 mm |
| IA-026 | 45,1 mm | 50,0 mm |
| IA-030 | 49,1 mm | 50,0 mm |
| | | |
| | | |
| | | |
| | | |
| | | |
| | | |
| | | |
| | | |

# Fig.11

## INPUT

| Manuelle Auswahl: | Abkürzung | Wert |
|---|---|---|
| Zahnlinien | | VITAPAN PLUS / 2 und VITA LINGOFORM |
| Frontzahnform | | T (Dreieckig) |
| Okklusionskonzept | | Bukkalkontakte |
| Nasenbreite | n | 38,0 mm |

| Landmarken in CAD-Programm | Abkürzung | Koordinaten (mm) | | |
|---|---|---|---|---|
| | | x | y | z |
| OK Tuber rechts | tr | 30 | 1 | 20 |
| OK Tuber links | tl | -29 | 2 | 21 |
| OK Papilla incisiva | pi | 0 | 70 | 20 |
| UK Retromolares Dreieck rechts | rdr | 27 | 0 | 3 |
| UK Retromolares Dreieck links | rdl | -27 | 0 | 4 |
| UK tiefste Stelle Mundvorhof rechts | mvr | 3 | 82 | -4 |
| UK tiefste Stelle Mundvorhof links | mvl | -4 | 81 | -4 |

## OUTPUT

| Output | Abkürzung | | |
|---|---|---|---|
| Zahnlinien | | VITAPAN PLUS / 2 und VITA LINGOFORM | |
| Frontzahnform | | T (Dreieckig) | |
| Okklusionskonzept | | Bukkalkontakte | |
| Garniturmaß Frontzähne | g | von 43,6 mm | bis 47,6 mm |
| Transversalmaß der Aufstellung | tma | von 47,1 mm | bis 50,1 mm |
| Skeletale Klasse | | Klasse 1 | |
| Bissart im Seitenzahnbereich | | Normalbiss | |

| Geeignete Aufstellungen | Garniturmaß | Transversalmaß |
|---|---|---|
| IA-078 | 45,1 mm | 50,0 mm |
| IA-082 | 47,3 mm | 50,0 mm |
| | | |
| | | |
| | | |
| | | |
| | | |
| | | |
| | | |
| | | |
| | | |
| | | |

# Fig.12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010151417 A1
- WO 2011066895 A1
- EP 1864627 A2
- EP 1444965 A2
- WO 2015078701 A